# EUROPEAN PATENT APPLICATION

(11) **EP 4 299 598 A1**
(43) Date of publication of application: **03.01.2024**
(21) Application number: 22781612.1
(22) Date of filing: 30.03.2022
(51) Int. Cl.: C08B 37/00, C08L 5/06, C08J 3/075, A61K 47/36, A61K 9/70, A61K 9/06, A61P 1/02

(54) **PECTIN MODIFIED WITH GALLOL DERIVATIVE AND USE THEREOF**

(30) Priority: 31.03.2021 KR 20210042285; 29.03.2022 KR 20220038786
(71) Applicant: University-Industry Foundation(UIF), Yonsei University, Seoul 03722 (KR); Cellartgen Inc., Seoul 03722 (KR)
(72) Inventor: CHO, Seung Woo, Seoul 03722 (KR); CHOI, Soo Jeong, Seoul 04731 (KR); HWANG, Yong Soon, Seoul 03722 (KR); JEON, Ji Hoon, Seoul (KR)
(74) Representative: Manitz Finsterwald Patent- und Rechtsanwaltspartnerschaft mbB
(86) International application number: PCT/KR2022/004512
(87) International publication number: WO 2022/211493

(57) **Abstract**

The present invention is intended to provide a hydrogel that utilizes the naturally occurring substance pectin to exhibit an equivalent or greater effect. Specifically, the present application is intended to provide a hydrogel or a patch which is stable, has strong adhesive strength, is advantageous for drug delivery, can be used for sustained treatment, has extremely low cytotoxicity, and is unlikely to induce an immune response, and thus can be used on various body parts, in particular the oral cavity. In addition, the present invention is intended to provide a hydrogel patch which employs a freezing method prior to crosslinking, and thus exhibits superior adhesion to patches employing a post-crosslinking freezing method.

## Description

### TECHNICAL FIELD

The present disclosure relates to pectin modified with a gallol derivative and a use thereof.

### BACKGROUND

Globally, the market related to functional medical materials for medical treatment, such as drug delivery, cell transplantation and hemostasis, and suturing is growing very rapidly. According to statistical data, the controlled release drug delivery market is expected to grow at an average annual rate of 13.8% until 2025 due to the growing trend of R&D and needs in elderly and pediatric patient groups. Also, the global stem cell market is expected to grow rapidly from $62.8 billion in 2017 to $394.4 billion in 2025.

Based on this expectation, the demand for materials for drug delivery and cell transplantation is expected to surge. Further, the hemostatic and suture material market is estimated to grow to 4.5 trillion won by 2024 and is expected to further grow in the future.

As needs for biomaterials with adhesion to the surface of various materials or biological tissue are required for stable drug delivery and cell transplantation into the living body, hemostasis of bleeding site, and suturing of wound tissue, various types of adhesive hydrogels are being developed and some are being applied to clinical trials.

The oral cavity generally refers to the area from the lips to the starting point of the throat, and is lined by the mucous membrane. The oral mucous membrane serves to maintain homeostasis of the human body and provide primary defense against external infection, and also serves as the pathway through which nutrients and drugs are absorbed. Defects in the mucous membrane can cause organ function loss due to bacterial infection and drying of submucosal tissues. Diseases occurring in the oral cavity include oral cancer, stomatitis, vitiligo, hand-foot-and-mouth disease, oral tuberculosis, oral candidiasis, and periodontitis. Recently, oral mucositis has emerged as the most serious side in treating cancer patients. Also, infections related to oral mucositis may cause systemic sepsis in a state in which immunity is lowered due to chemotherapy, and may result in death. Such intractable oral ulcers cause pain, decrease nutritional intake, and increase a need for intravenous nutrition and thus degrade the patients' quality of life. In particular, intractable oral ulcers may act as high-risk factors for sepsis in cancer patients with neutropenia, which leads to death in 10% of patients with severe oral mucositis. Further, the cancer recurrence rate is increasing due to reduced doses of planned chemotherapy and radiotherapy, or induction of treatment discontinuation. Furthermore, when oral mucositis develops, additional treatment and procedures, such as prescription of opioid analgesics to control pain, prescription of antibiotics to prevent infection, and intravenous nutrition, are required. Therefore, the development of oral ulcer during chemotherapy may cause the aggregate medical expense to more than double. The number of oral mucositis patients in Korea has increased by more than 30% and the amount of medical expense has increased by about 70% over the past 6 years.

As described above, there are various diseases, such as stomatitis, which may occur in the oral cavity, and the incidence rate is also increasing. However, it is difficult to introduce drugs into the oral cavity by using a patch or the like because the drugs cannot last for a long time due to the flow of saliva in the oral cavity and many curves are present in the oral cavity. Therefore, to increase the efficiency in treating a wide range of oral diseases, there is an urgent need to develop a mucoadhesive patch capable of effectively adhering to the surface of the oral mucous membrane with many curves and continuously releasing a drug for a long time.

In order to solve this problem, Korean Patent No. 10-1942220 provides a chitosan membrane comprising a catechol group and a preparation method thereof. This prior art invention can effectively adhere to the surface of the oral mucous membrane with many curves, collecting various drugs and continuously releasing the drugs for a long time.

Besides, many prior art documents such as Korean Patent Laid-open Publication No. 10-2018-0127634 have adopted a post-crosslinking freeze-drying method when a hydrogel is fabricated, which results in poor adhesion.

### DISCLOSURE OF THE INVENTION

### PROBLEMS TO BE SOLVED BY THE INVENTION

The present disclosure is conceived to provide a hydrogel that uses pectin existing in nature and exhibits the same or greater effect than Korean Patent No. 10-1942220 and Korean Patent Laid-open Publication No. 10-2018-0127634. Specifically, the present disclosure is conceived to provide a hydrogel or patch that can be used in various body parts, especially in the oral cavity, because it is stable, has strong adhesion, is advantageous for drug delivery, can be used for sustained treatment, has extremely low cytotoxicity, and is less likely to induce an immune response. Also, the present disclosure adopts a freezing method prior to crosslinking and thus provides a hydrogel patch having excellent adhesion compared to a hydrogel patch prepared by a post-crosslinking freezing method.

However, the problems to be solved by the invention are not limited to the above-described problems and should be construed as including all problems within a range that can be understood by a person with ordinary skill in the art.

### means for solving the problems

In order to solve the above-described problems, a first aspect of the present disclosure provides a pectin derivative modified with a gallol derivative (Pec-PG).

A second aspect of the present disclosure provides a hydrogel prepared by oxidizing the pectin derivative.

A third aspect of the present disclosure provides a patch including the pectin derivative.

fourth aspect of the present disclosure provides a method of preparing pectin modified with a gallol derivative, including a process of reacting pectin, EDC, NHS, and a gallol derivative in a predetermined ratio.

The above-described means for solving the problems are merely examples and should be construed as including all means within a range that can be understood by a person with ordinary skill in the art.

### EFFECTS OF THE INVENTION

Pectin modified with a gallol derivative according to the present disclosure controls a swelling ratio and a degradation ratio, enables a strong bond between a mucosal tissue and a gallol group, controls a drug delivery rate, exhibits excellent biocompatibility due to low cytotoxicity, and is less likely to induce an immune response in vivo, and, thus, it is stable as a hydrogel. Therefore, it can be applied to various body parts, especially to the oral cavity where adhesion is difficult.

### BRIEF DESCRIPTION OF THE DRAWINGS

**FIG. 1A** shows an example of a process of producing pectin modified with a gallol group, **FIG. 1B** shows the results of a test confirming whether a hydrogel is formed at each concentration of Pec-PG, and **FIG. 1C** shows the results of a test confirming a suitable concentration for spray formulation.
**FIG. 2A** and **FIG. 2B** are diagrams showing the results of analyzing hydrogel formation kinetics during crosslinking depending on the origin of pectin according to Example 2.
**FIG. 3A** and **FIG. 3B** are diagrams showing the results of analyzing a swelling ratio (**FIG. 3A**) and a degradation ratio (**FIG. 3B**) among physical properties of a gallol group-modified pectin hydrogel according to Example 3.
**FIG. 4A** and **FIG. 4B** show the internal structure of a hydrogel observed by scanning electron microscopy (SEM), hematoxylin & eosin (H&E) staining, and toluidine blue (TB) staining (**FIG**. **4A**), and show a pore diameter in the hydrogel measured with a porosimeter (**FIG. 4B**) according to Example 4.
**FIG. 5A** and **FIG. 5B** show the result of Fourier-transform infrared spectroscopy (FT-IR) (**FIG. 5A**) and the result of UV-vis spectrophotometry (**FIG. 5B****)** according to Example 5.
**FIG. 6A** and **FIG. 6B** show the results of measuring an elastic modulus of a hydrogel in a frequency sweep mode by using a rheometer according to Example 6.
**FIG. 7A** to **FIG. 7D** show the results of analyzing the reaction between Pec-PG and mucin on the surface of a mucosal layer by atomic force microscopy (AFM) **(****FIG. 7A** and **FIG. 7B****),** the result of UV-vis spectrophotometry (FIG. 7C), and the result of analyzing a zeta potential of a mixture of Pec-PG and mucin (**FIG. 7D**) according to Example 7.
**FIG. 8** shows the results of comparing the release pattern of a protein, bovine serum albumin (BSA), according to Example 8.
**FIG. 9A** and **FIG. 9B** show the cell viability measured by live/dead staining (**FIG. 9A**) and proliferation rates of stem cells cultured in Pec-PG hydrogels measured by MTT assay (**FIG. 9B**) according to Example 9.
**FIG. 10A** to **FIG. 10C** show the result of comparing the amount of TNF-α secretion between a Pec-PG hydrogel of the present disclosure and a control group (**FIG. 10A**) and the results of checking an immune response in mice by H&E staining (**FIG. 10B**) and TB staining (**FIG. 10C**) according to Example 10.
**FIG. 11A** and **FIG. 11B** show the result of comparing the body weight of the rat's main organs between a hydrogel of the present disclosure and a control group by conducting an oral administration test to evaluate the safety of the hydrogel of the present disclosure (**FIG. 10A**) and the result of histological analysis through H&E staining (**FIG. 11B**) according to Example 11.
**FIG. 12A** to **FIG. 12C** show the results of confirming the tissue adhesion of a hydrogel of the present disclosure in a pig tongue tissue **(****FIG. 12A****),** a living rat tongue tissue **(****FIG. 12B****),** and a rat tongue tissue coated with a hydrogel mixed with a fluorescent material **(****FIG. 12C****)** according to Example 12.
**FIG. 13A** to **FIG. 13D** show the results of a test confirming a moisture evaporation rate and a moisturizing function in a Petri dish **(****FIG. 13A****),** a sponge **(****FIG. 13B****),** and a living rat tongue tissue **(****FIG. 13C** and **FIG. 13D****)** according to Example 13.
**FIG. 14A** to **FIG. 14C** are diagrams showing the results of analyzing a swelling ratio (**FIG. 14B**) and a degradation ratio **(****FIG. 14C****)** of a patch **(****FIG. 14A****)** of the present disclosure according to Example 14.
**FIG. 15A** and **FIG. 15B** show a pore diameter in a patch measured with a porosimeter **(****FIG. 15A****),** and compares a pore diameter between a hydrogel and a patch according to Example 15.
**FIG. 16A** and **FIG. 16B** show the results of measuring an elastic modulus of a hydrogel patch by using a rheometer according to Example 16.
**FIG. 17A** and **FIG. 17B** show the results of comparing the tissue adhesion of hydrogel patches by a tack test according to Example 17.
**FIG. 18** shows the result of checking a drug release pattern by HPLC according to Example 18.
**FIG. 19A** to **FIG. 19C** show the results of analyzing an antioxidant function according to Example 19.

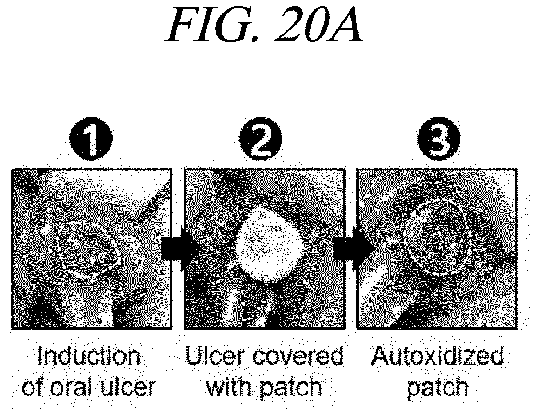

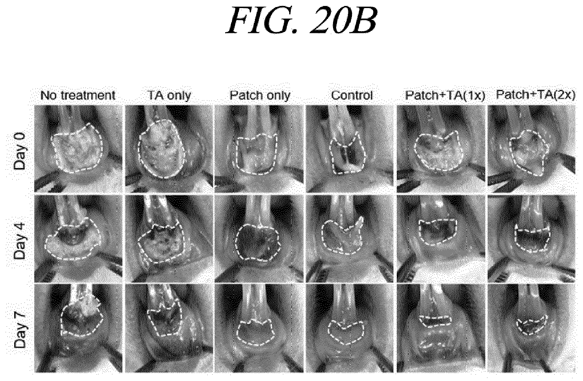

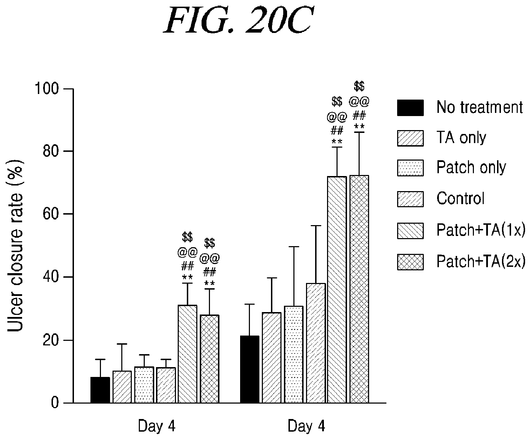

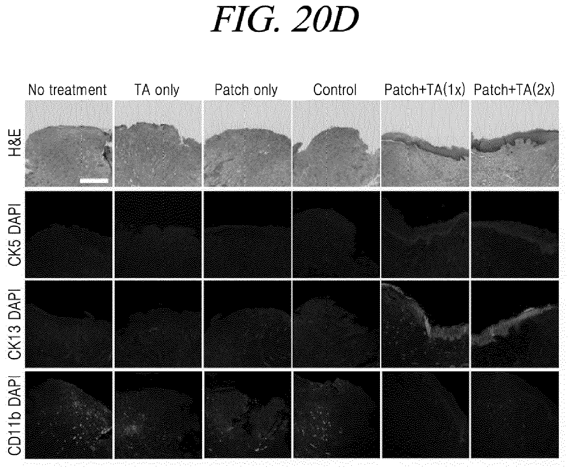

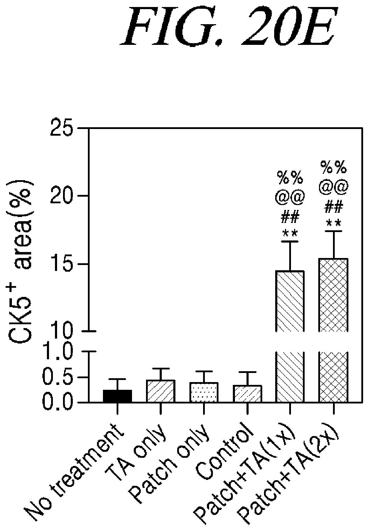

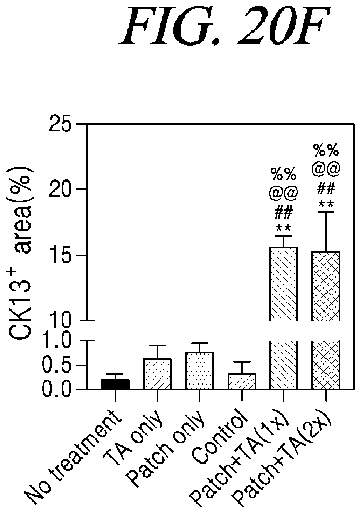

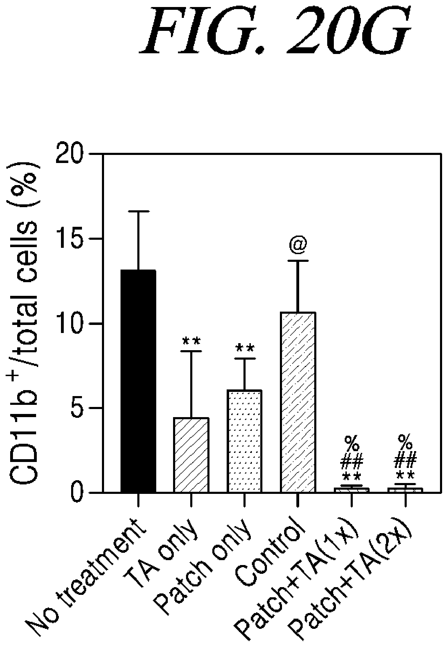

**FIG. 20A** to **FIG. 20G** show the result of confirming the effects of gum regeneration and inflammation treatment in rats **(****FIG. 20A****),** the results of confirming the area of a damage site and the effect of tissue regeneration **(****FIG. 20B** and **FIG. 20C****),** the results of H&E staining, immunostaining for epithelial tissue markers (cytokeratin 5; CKS, cytokeratin 13; CK13) and staining image-based quantitative analysis **(****FIG. 20D** to **FIG. 20F****),** and the result of confirming the number of inflammatory cells by immunostaining for CD11b, a leukocyte marker **(****FIG. 20G****)** according to Example 20.
**FIG. 21A** and **FIG. 21B** show the results of analyzing the reactivity and interaction between mucin protein and each derivative by atomic force microscopy (AFM), and **FIG. 21C** shows the result of comparing mucoadhesion of a Pec-PG hydrogel patch, a HA-PG hydrogel patch and a Chi-CA hydrogel patch in a mucosal tissue of the pig gum by a tack test based on **FIG. 21A** and **FIG. 21B****.** according to Comparative Example 1.
**FIG. 22** shows the result of comparing a gallol group-modified pectin hydrogel patch according to Comparative Example 2 with one prepared by a prior art method.

### BEST MODE FOR CARRYING OUT THE INVENTION

Hereinafter, embodiments will be described in detail with reference to the accompanying drawings so that the present disclosure may be readily implemented by a person with ordinary skill in the art. However, it is to be noted that the present disclosure is not limited to the embodiments but can be embodied in various other ways. In the drawings, parts irrelevant to the description are omitted for the simplicity of explanation, and like reference numerals denote like parts through the whole document.

Through the whole document, the term "on" that is used to designate a position of one element with respect to another element includes both a case that the one element is adjacent to the other element and a case that any other element exists between these two elements.

Further, through the whole document, the term "comprises or includes" and/or "comprising or including" used in the document means that one or more other components, steps, operation and/or existence or addition of elements are not excluded in addition to the described components, steps, operation and/or elements unless context dictates otherwise.

Through the whole document, the term "about or approximately" or "substantially" is intended to have meanings close to numerical values or ranges specified with an allowable error and intended to prevent accurate or absolute numerical values disclosed for understanding of the present disclosure from being illegally or unfairly used by any unconscionable third party. Through the whole document, the term "step of" does not mean "step for".

Through the whole document, the term "combination(s) of" included in Markush type description means mixture or combination of one or more components, steps, operations and/or elements selected from a group consisting of components, steps, operation and/or elements described in Markush type and thereby means that the disclosure includes one or more components, steps, operations and/or elements selected from the Markush group.

Through the whole document, a phrase in the form "A and/or B" means "A or B, or A and B".

Through the whole document, all examples of a material are merely examples and the material is not limited a specific example.

Through the whole document, the term "pectin" refers to a hydrated gel surrounding the cellulose-hemicellulose network and is a polysaccharide whose main component is galacturonic acid, an oxide of galactose. Pectin acts as a hydrophilic filler to suppress aggregation and collapse of the network and plays a role in determining the porosity of the polymer in the cell wall. The major part consists of α-1,4 bonds in D-galacturonic acid units.

Through the whole document, the term "gallol group derivative" refers to a compound having a pyrogallol group represented by C₆H₃(OH)₃ or a structure of Chemical Formula 1.

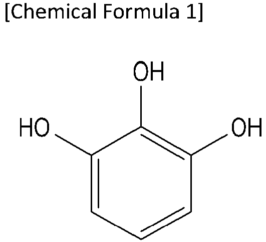

Through the whole document, term "stomatitis" refers to inflammation that occurs in the mouth and generally refers to inflammatory diseases occurring in the mucous membranes (tongue, gums, inside of the lips and cheeks, etc.) in the oral cavity. The causes of stomatitis include bacterial, viral, and fungal infection, and when inflammation is severe, blisters, ulcers and the like may occur. Stomatitis may include oral mucositis, oral cancer, stomatitis, vitiligo, hand-foot-and-mouth disease, oral tuberculosis, oral candidiasis, mucosal wounds, periodontitis, aphthous ulcer, Behcet's syndrome, lichen planus, herpetic stomatitis, etc., and may be construed as including all inflammations occurring in the oral cavity.

Through the whole document, the term "patch" refers to a membrane that can be attached to the mucosal surface in the oral cavity, and may include a therapeutic drug to be used for disease treatment because it can release the drug over a long period of time. When the patch does not include a drug, it can also be used for treatment by adhering to the surface of a wound within a tissue and inhibiting additional infection of the wound site.

Through the whole document, the term "adhesion" may be mainly due to bonding between a mucosal tissue and a phenol group derivative. Possible bonds include covalent bonds and various physical non-covalent bonds, such as hydrogen bonds, hydrophobic interactions, and pi-pi interactions.

A first aspect of the present disclosure provides a pectin derivative modified with a gallol derivative (Pec-PG). The Pec-PG is expected to be applicable to various biotechnology fields due to excellent tissue adhesion of the gallol group. In particular, the Pec-PG is highly effective as artificial saliva or oral drug delivery matrix to which adhesiveness to the mucous membrane is important. Also, due to natural oxidation of the gallol group, the Pec-PG in a solution form can be sprayed or coated to the oral cavity, and, thus, it can be coated or attached to hard-to-reach sites of the oral cavity.

Herein, the gallol group derivative may be one selected from the group consisting of 5-hydroxydopamine, tannic acid, gallic acid, epigallocatechin, epicatechin gallate, epigallocatechin gallate, 2,3,4-trihydroxybenzaldehyde, 2,3,4-trihydroxybenzoic acid, 3,4,5-trihydroxybenzaldehyde, 3,4,5-trihydroxybenzamide, 5-tert-butylpyrogallol, and 5-methylpyrogallol.

A second aspect of the present disclosure provides a hydrogel prepared by oxidizing the pectin derivative according to the present disclosure. The hydrogel of the present disclosure has all properties of the first aspect. In particular, a sustained release of the loaded drug is possible due to excellent reactivity between the oxidized gallol group and various nucleophilic functional groups. Thus, the hydrogel can be applied to various diseases and sites depending on the formulation.

The oxidation of the hydrogel may be performed by adding an oxidizing agent, an enzyme, or a pH adjustor. The oxidizing agent may be periodate salt or hydrogen peroxide, and the enzyme may be one selected from the group consisting of peroxidase, horseradish peroxidase, and tyrosinase. The pH adjustor may be one selected from the group consisting of sodium hydroxide, lithium hydroxide, potassium hydroxide, rubidium hydroxide, cesium hydroxide, magnesium hydroxide, calcium hydroxide, strontium hydroxide, and barium hydroxide.

In addition, the oxidation of the hydrogel is also possible through natural oxidation. Accordingly, the Pec-PG in a solution form can be sprayed or coated to the oral cavity, and, thus, it can be coated or attached to hard-to-reach sites of the oral cavity.

An application site of the hydrogel according to the present disclosure may include oral mucosa, nasal mucosa, ophthalmic mucosa, gastric mucosa, intestinal mucosa, bronchial mucosa, heart, lung, or urinary tract, and, thus, the hydrogel according to the present disclosure can be used to treat mucosal tissue diseases. In particular, the hydrogel according to the present disclosure can be effective in treating mucosal tissue diseases including oral mucositis, oral cancer, stomatitis, vitiligo, hand-foot-and-mouth disease, oral tuberculosis, oral candidiasis, mucosal wounds, periodontitis, aphthous ulcer, Behcet's syndrome, lichen planus, or herpetic stomatitis.

Also, the hydrogel according to the present disclosure can be used to control and deliver a drug in vivo. Herein, the drug may be encapsulated or supported in the hydrogel, and the drug may include one or more selected from the group consisting of DNA, mRNA, siRNA, miRNA, antisense oligonucleotide, antihistamine, proteins (growth factors), corticosteroid-based steroids, immune cell activators, anticancer agents, therapeutic antibodies, antibiotics, antibacterial agents, antiviral agents, anti-inflammatory agents, protein drugs, growth factors, cytokines, peptide drugs, and anesthetics.

The anticancer agent may include one or more selected from the group consisting of doxorubicin, daunomycin, epirubicin, anthracyclines, such as idarubicin, paclitaxel, docetaxel, cabazitaxel, taxanes, such as tecetaxel, curcumin, campotecin, berberine, evodiamine, matrine, piperine, sanguinarine, tetrandrine, talicarpine, alkaloids, such as ellipticine, vinblastine, vincristine, vindesine, vinca alkaloids, such as vinorelbine, cisplatin, carboplatin, platinums, such as oxaliplatin, 5-fluorouracil, capecitabine, methotrexate, antimetabolites, such as gemcitabine, irinotecan, topotecan, etoposide, teniposide, etoposide, topoisomerase inhibitors, such as amsacrine, bleomycin, actinomycin, mitomycin, antitumor antibiotics, such as mitoxantrone, cyclophosphamide, mechlorethamine, chlorambucil, melphalan, alkylating agents, such as nitrosourea, azacitidine, azathioprine, cytarabine, and doxifluridine.

A third aspect of the present disclosure provides a patch including the pectin derivative. The patch according to the present disclosure can be fabricated by thinly freezing the Pec-PG solution and then freeze-drying it, and drug delivery is possible by loading a desired drug on a hydrogel or patch. In particular, according to the existing prior art documents, such as Korean Patent Laid-open Publication No. 10-2018-0127634, a patch is fabricated mainly by a post-crosslinking freeze-drying method, but the present disclosure adopts a freeze-drying method prior to crosslinking. Due to the difference between these two methods, the patch of the present disclosure may have remarkably improved adhesion compared to the existing patch.

Also, the Pec-PG hydrogel patch loaded with the drug is very user-friendly and easy to apply in vivo.

A fourth aspect of the present disclosure provides a method of preparing pectin modified with a gallol derivative.

The method may include a process of reacting pectin, EDC, NHS, and a gallol derivative in a predetermined ratio, and the ratio of pectin:EDC:NHS:PG may be 1:2:2:2, but is not limited thereto.

The common features for the first to fourth aspects of the present disclosure may equally apply to each aspect.

Hereinafter, embodiments and examples of the present disclosure will be described in detail with reference to the accompanying drawings. However, the present disclosure may not be limited to the following embodiments, examples, and drawings.

### Example 1. Synthesis of gallol group-modified pectin derivative, fabrication of hydrogel and confirmation of suitable concentration

A pectin derivative was synthesized by modifying pectin (Pec), which often used as a food additive or food supplement, with a gallol group (PG), and a Pec-PG hydrogel was fabricated based on the pectin derivative.

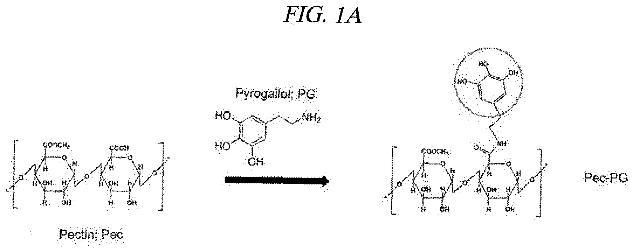

For a gallol group, 5-hydroxydopamine was used, and a pectin derivative (Pec-PG) was synthesized in a molar ratio of pectin:EDC:NHS:PG of 1:2:2:2 through a chemical reaction of 1-(3-dimethylaminopropyl)-3-ethylcarbodiimide hydrochloride (EDC)/N-hydroxysuccinimide (NHS) (see FIG. 1A).

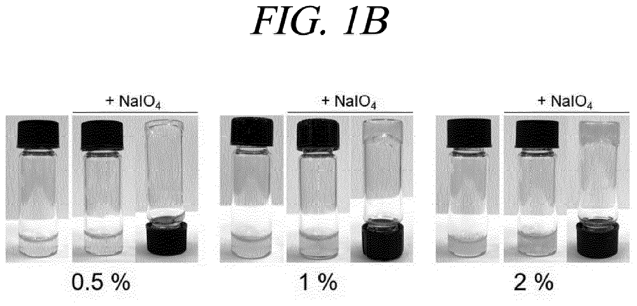

A hydrogel was not formed when the Pec-PG at a concentration of less than 1% (w/v) was treated with 4.5 mg/ml NalO₄ as an oxidizing agent, and at a concentration of 1%, a hydrogel having very weak physical properties was formed and was easily collapsed even by slight shaking. Since a hydrogel was stably formed at a concentration of 2% (w/v) or more, the Pec-PG at a concentration of 2% (w/v) or more was used in the examples of the present disclosure (see **FIG. 1B**). However, this does not mean to limit the concentration of the Pec-PG of the present disclosure.

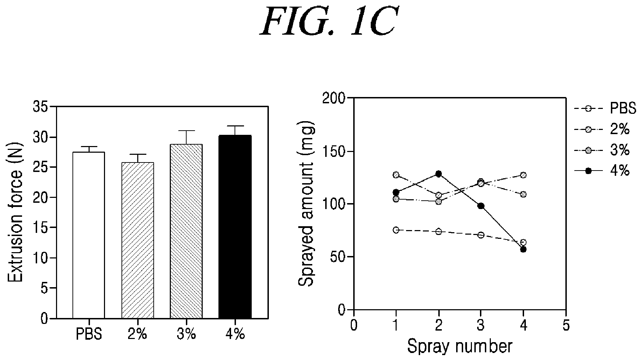

Also, to confirm a suitable concentration for spray formulation, a test was conducted by spraying a Pec-PG solution at various concentrations of the Pec-PG through a spray nozzle. As the concentration of the Pec-PG increased, the amount of force required for spraying increased (see **FIG. 1C****;** left).

As the concentration of the Pec-PG increased, the amount of the Pec-PG sprayed was varied for each time. In particular, when the Pec-PG at a concentration of 4% was sprayed repeatedly at least 3 times, the amount of the Pec-PG sprayed greatly decreased. This suggests that the ease of spraying gradually decreases as the concentration of the Pec-PG increases. (see **FIG. 1C**; right).

Therefore, it was confirmed that the Pec-PG at a concentration of 2% was suitable for spray formulation. This does not mean that the Pec-PG at the other concentrations cannot be used, but only that a concentration of 2% is suitable for spray formulation.

### Example 2. Analysis of hydrogel formation kinetics during crosslinking depending on origin of pectin

Analysis of hydrogel formation kinetics was performed during crosslinking depending on the origin of pectin. Gelation kinetics was compared when citrus peel-derived pectin and apple-derived pectin were modified with the gallol group (PG) through EDC/NHS reaction and a hydrogel was formed by inducing crosslinking of the synthesized pectin derivatives.

A process of forming a hydrogel when crosslinking was induced by mixing 4.5 mg/ml NalO₄ as an oxidizing agent in a 2% (w/v) citrus Pec-PG pre-gel solution and a 2% (w/v) apple Pec-PG pre-gel solution was analyzed.

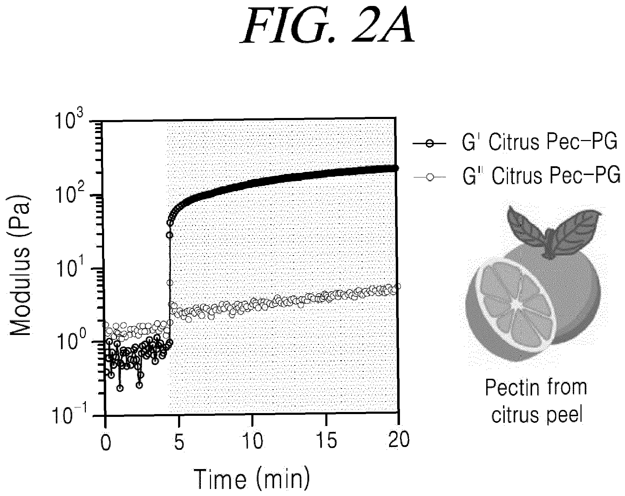

It was confirmed that a loss modulus (G") value was higher than a storage modulus (G') value in the 2% (w/v) citrus Pec-PG pre-gel solution and when the oxidizing agent was mixed for crosslinking, the G' value was higher than the G" value. This means that the citrus peel-derived Pec-PG can stably form a hydrogel (see **FIG. 2A**).

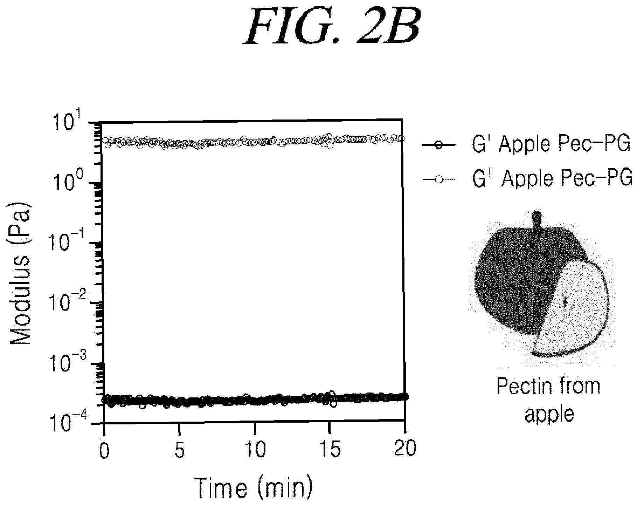

The G" value was maintained higher than the G' value in the 2% (w/v) apple Pec-PG pre-gel solution, and even when the oxidizing agent was mixed for crosslinking, no numerical change was observed. This means that it is difficult for the apple-derived Pec-PG to form a hydrogel (see **FIG. 2B**).

That is, pectin varies in structure depending on the origin, and, thus, even when pectin is modified with a gallol group, a hydrogel cannot be formed. Also, it was confirmed that the degree of esterification of pectin and the amount of galacturonic acid having a modification functional group (COOH group) can affect the synthesis of a Pec-PG derivative. Actually, citrus peel-derived pectin contains more than 70% of galacturonic acid and thus has sufficient functional groups for gallol group modification. Therefore, there is no problem in synthesizing a Pec-PG derivative and forming a hydrogel. Also, apple-derived pectin has a relatively low ratio of galacturonic acid and is esterified to a great extent. Therefore, it seems difficult to synthesize a Pec-PG derivatives and form a hydrogel.

### Example 3. Analysis of physical properties of gallol group-modified pectin hydrogel

Hydrogels were fabricated by mixing 4.5 mg/ml NalO₄ as an oxidizing agent in Pec-PG pre-gel solutions at two commonly used concentrations (2% (w/v), 4% (w/v)) to induce crosslinking. Then, the swelling ratios of the respective hydrogels were measured.

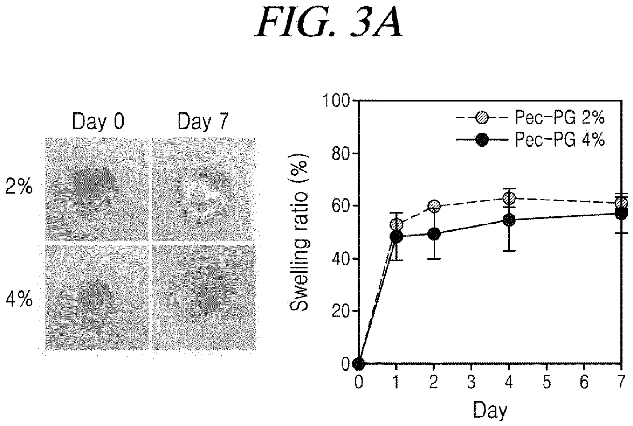

When kept under physiological conditions (37°C in PBS buffer) for 7 days, the 2% (w/v) hydrogel showed a slightly higher swelling ratio than the 4% (w/v) hydrogel until day 4 (see **FIG. 3A**). This suggests that as the concentration decreases, the degree of crosslinking of the polymer decreases and the swelling ratio increases.

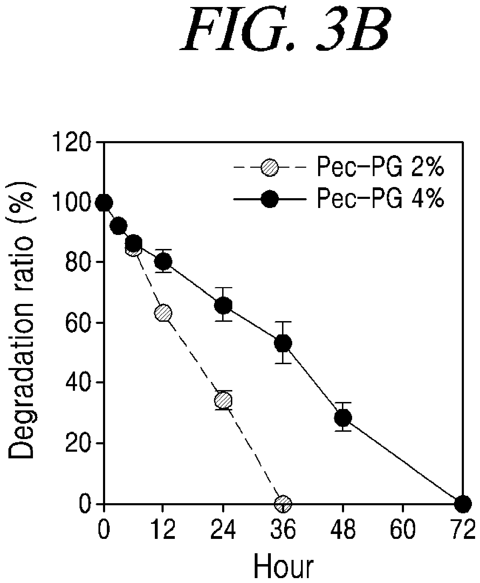

To compare the degradation pattern of the Pec-PG hydrogel, the hydrogel was treated with a pectinase. When treated with the same concentration of the pectinase (1 U/sample), the 4% (w/v) hydrogel exhibited a high degree of crosslinking of gallol groups. Therefore, it was confirmed that the hydrogel at a higher concentration was degraded later (see **FIG. 3B**).

That is, the physical properties, such as swelling ratio and degradation behavior, of the Pec-PG hydrogel can be controlled by controlling the concentration of the Pec-PG.

### Example 4. Analysis of internal structure of gallol group-modified pectin hydrogel

A hydrogel was fabricated by mixing 4.5 mg/ml NalO₄ as an oxidizing agent in the 2% (w/v) Pec-PG pre-gel solution.

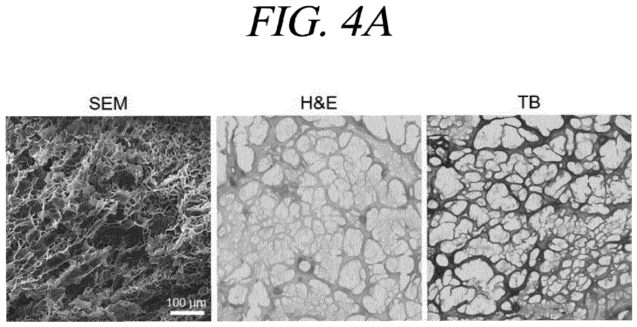

When the internal structure of the hydrogel was observed by scanning electron microscopy (SEM), hematoxylin & eosin (H&E) staining, and toluidine blue (TB) staining, the hydrogel had a dense porous structure at the micro level (see **FIG. 4A**).

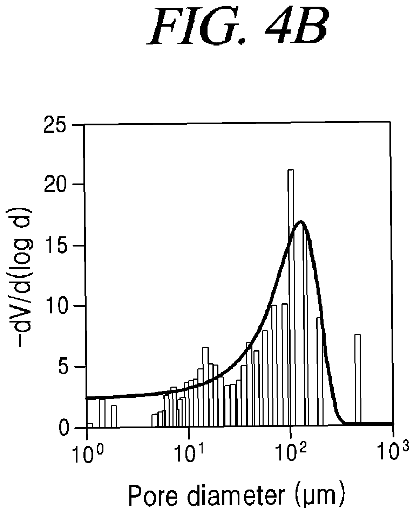

When a pore diameter in the hydrogel was measured with a porosimeter, a large number of pores having a diameter of about 100 µm were distributed (see **FIG. 4B**).

Since the hydrogel according to the present disclosure has such a porous structure, it provides a favorable environment for cell adhesion and proliferation and may be effective in loading and releasing a drug.

### Example 5. Chemical characterization of gallol group-modified pectin hydrogel

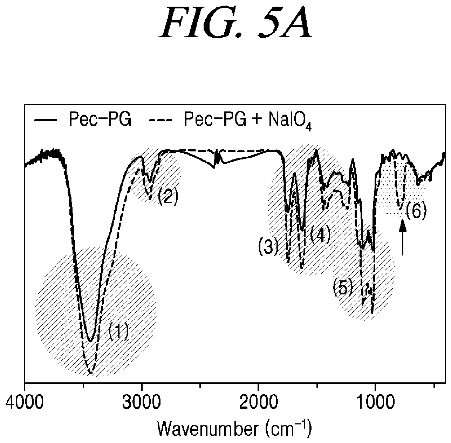

The chemical structure of the Pec-PG (1 mg/ml) before (Pec-PG) and after (Pec-PG + NalOₐ) oxidation was analyzed by Fourier-transform infrared spectroscopy (FT-IR) (see **FIG. 5A**).

All the peaks around 3435 cm⁻¹ representing an O-H group were observed.

All the peaks around 2925 cm⁻¹ representing a C-H stretching group were observed.

All the peaks around 1743 cm⁻¹ representing an ester carbonyl stretching group were observed.

All the peaks around 1620 cm⁻¹ and around 1440 cm⁻¹ representing a carboxylate ion stretching group were observed.

All the peaks around 1100 cm⁻¹ peak and around 1020 cm⁻¹ representing a galacturonan backbone were observed.

The 784 cm⁻¹ peak indicating the formation of biphenol and biphenyl ether was observed only in the oxidized Pec-PG.

Accordingly, crosslinking between oxidized gallol groups (PG) was observed only in the Pec-PG treated with the oxidizing agent.

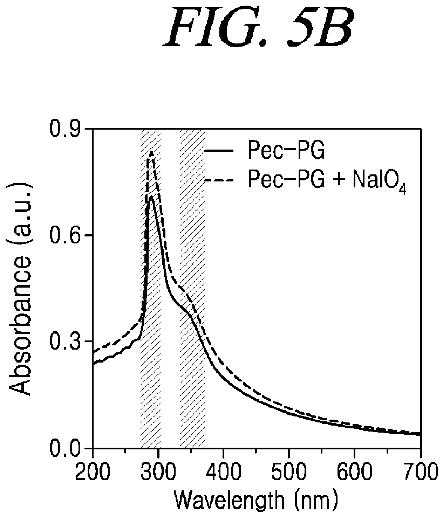

A chemical reaction occurring after oxidation by adding an oxidizing agent (NalO₄) to the Pec-PG solution was analyzed by UV-vis spectrophotometry (see **FIG. 5B**).

After the oxidizing agent was added, the 280 nm and 350 nm peaks increased. Accordingly, it was confirmed that as the gallol group (PG) was oxidized, semi-quinone and phenoxyl radical were formed and crosslinking occurred.

### Example 6. Analysis of mechanical properties of gallol-modified pectin hydrogel

Hydrogels were fabricated by mixing 4.5 mg/ml NalO₄ as an oxidizing agent in Pec-PG pre-gel solutions at two different concentrations (2% (w/v), 4% (w/v)).

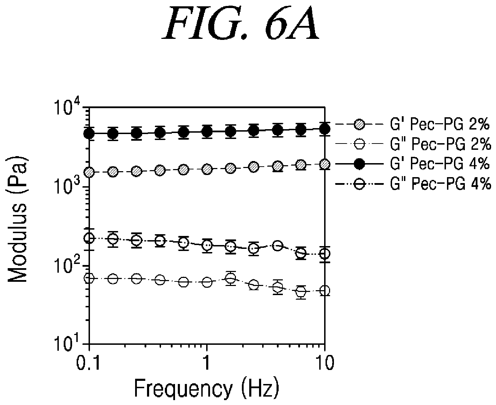

The elastic modulus of each hydrogel was measured in a frequency sweep mode by using a rheometer (see **FIG. 6A**).

It was confirmed that a stable polymer network structure was formed in each hydrogel by confirming that the G' value was higher than the G" value in both the 2% (w/v) and 4% (w/v) Pec-PG hydrogels.

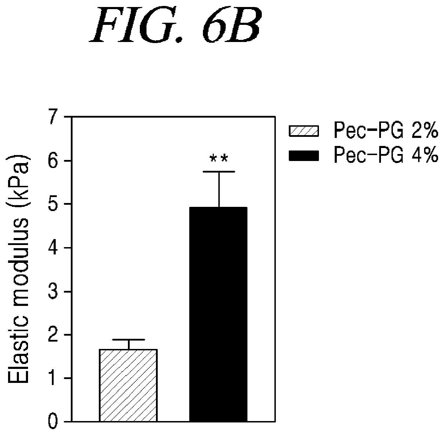

The average elastic modulus of the 2% (w/v) Pec-PG hydrogel was about 1.7 kPa, whereas the average elastic modulus of the 4% (w/v) Pec-PG hydrogel was about 4.9 kPa, which confirmed that the elastic modulus increases in proportion to the concentration of the Pec-PG (see **FIG. 6B**).

That is, the mechanical properties of the Pec-PG hydrogel can be controlled by controlling the concentration of the Pec-PG.

### Example 7. Analysis of adhesion to surface of mucous membrane of gallol group-modified pectin

A reaction pattern between the Pec-PG and mucin on the surface of the mucosal layer was analyzed by atomic force microscopy (AFM).

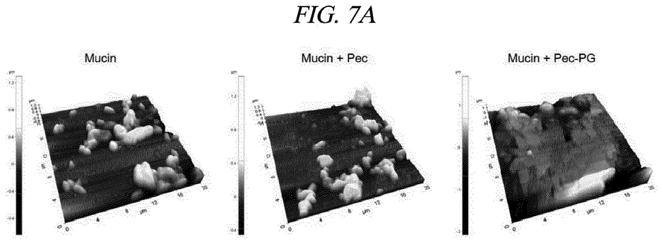

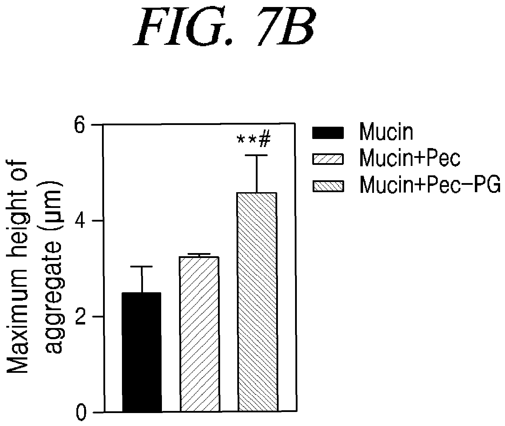

With mucin itself or a mixture of mucin and pectin (2 mg/ml Pectin), the formation of small aggregates was observed on the surface, and with a mixture of mucin and 2 mg/ml Pec-PG, significant large aggregates were formed in layers on the surface (see **FIG. 7A** and **FIG. 7B****).** It seems that adhesion is achieved through strong bonding reactions and interactions between the gallol group of the Pec-PG and the nucleophilic functional groups of mucin present in the mucosal tissue.

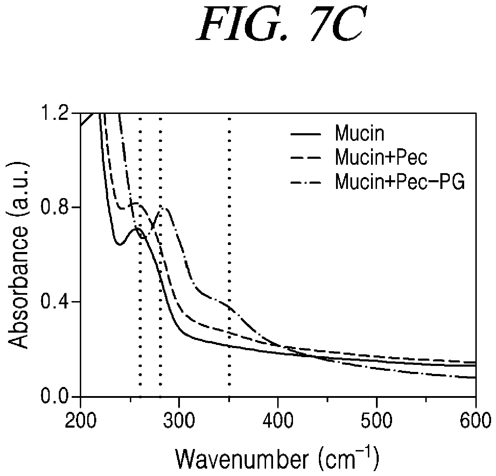

Mucin present on the surface of the mucosal layer was mixed with Pec-PG solution, and a chemical reaction between mucin and the gallol group (PG) was analyzed by UV-vis spectrophotometry. When 0.5 mg/ml Pec-PG was mixed with 0.5 mg/ml mucin, peaks around 280 nm and around 350 nm were increased. Accordingly, it was confirmed that strong covalent bonding occurred between the gallol group and the nucleophilic functional groups of mucin present in the mucosal tissue (see **FIG. 7C**).

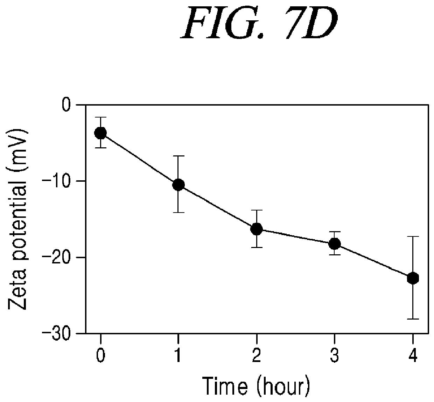

A zeta potential was analyzed to analyze the reaction between the Pec-PG and mucin on the surface of the mucosal layer over time. As time passed after mucin was mixed with 2% (w/v) Pec-PG, a zeta potential of the reactant became more and more negatively charged due to strong bonding reactions and interactions between the gallol group of the Pec-PG and the nucleophilic functional groups of mucin. This means that a strong covalent bond is formed between the gallol group of the Pec-PG and the nucleophilic functional groups of mucin (see **FIG. 7D**)**.**

Therefore, the Pec-PG according to the present disclosure can stably adhere to the mucosal tissue.

### Example 8. Analysis of drug release pattern of gallol group-modified pectin hydrogel

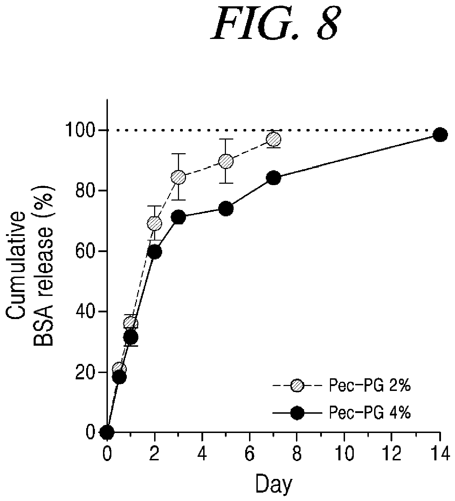

To verify sustained drug release delivery of the Pec-PG hydrogel, the release pattern of a protein, bovine serum albumin (BSA), was compared between the following two groups under physiological conditions (37 °C in PBS buffer) (see **FIG. 8**).
1) 2% (w/v) Pec-PG hydrogel loaded with BSA (Pec-PG 2%)
2) 4% (w/v) Pec-PG loaded with BSA (Pec-PG 4%)

When the drug release pattern was checked by BCA assay, a sustained drug release behavior of the Pec-PG hydrogel was observed due to strong bonding between the gallol group and various nucleophilic functional groups of the protein.

Since the 2% Pec-PG hydrogel with a low concentration had a slightly higher degradation rate, BSA was released faster. The 4% Pec-PG hydrogel released BSA over a longer time than the 2% Pec-PG hydrogel.

Therefore, the Pec-PG hydrogel loaded with a drug can be used for effective drug delivery to a site where effective delivery is difficult, such as an oral disease site. Also, it is expected that a continuous treatment for each tissue can be induced by adjusting the concentration as needed.

### Example 9. Evaluation of cytotoxicity of gallol group-modified pectin hydrogel

Human adipose-derived stem cells (hADSC) were encapsulated in the Pec-PG pre-gel solution, and crosslinking was induced by adding 4.5 mg/ml NalO₄ as an oxidizing agent, followed by three-dimensional culture of the stem cells in the Pec-PG hydrogel.

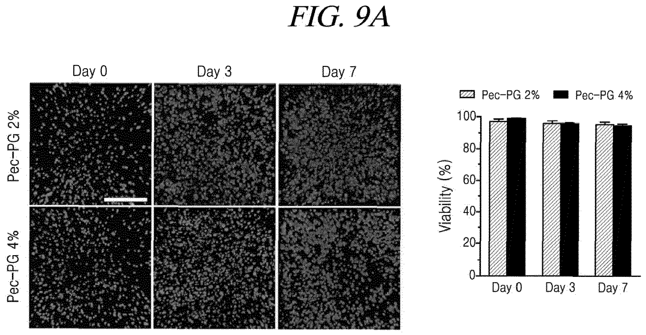

The cell viability was confirmed by live/dead staining while culturing the cells for 7 days. A high cell viability of 94% or more was observed throughout the culture period. Thus, it was confirmed that the Pec-PG hydrogel has no cytotoxicity and has excellent biocompatibility (see **FIG. 9A**).

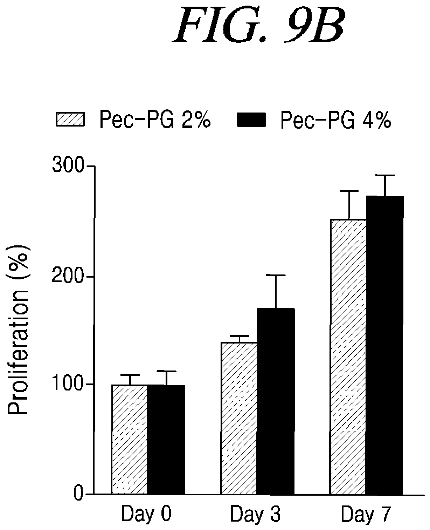

The proliferation rates of the stem cells cultured in the Pec-PG hydrogel was confirmed by MTT assay. It was confirmed that the stem cells proliferated normally during culture for 7 days (see FIG. 9B).

Therefore, the Pec-PG hydrogel does not show cytotoxicity and has excellent biocompatibility.

### Example 10. Evaluation of immune response of gallol group-modified pectin hydrogel

Through co-culture of macrophages and the Pec-PG hydrogel, TNF-α (tumor necrosis factor-a), a pro-inflammatory cytokine secreted from the macrophages, was measured by ELISA to confirm the degree of induction of immune response.

The macrophages were cultured on the bottom of a well-plate, a trans-well was placed thereon and then, the 2% (w/v) Pec-PG hydrogel was placed on the trans-well, followed by co-culture in a state where the cells were only affected by components eluted from the hydrogel without a direct contact. As a control group, lipopolysaccharides (LPS) were used.

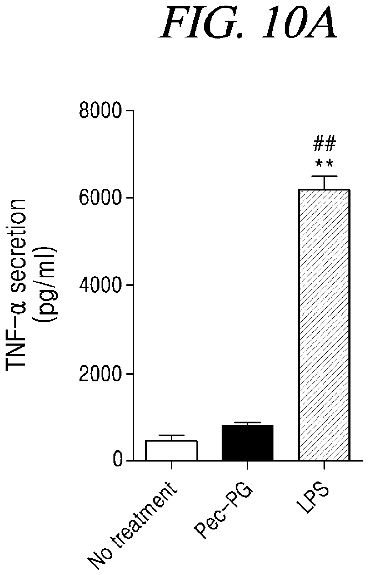

**As** a result, when the macrophages were co-cultured with the Pec-PG hydrogel, the amount of TNF-α secretion was similar to that of No treatment group, but remarkably different from that of the control group (see **FIG. 10A**).

In another test, the 2% (w/v) Pec-PG pre-gel solution was injected subcutaneously in mice without oxidizing agent treatment, crosslinking was induced through natural oxidation under an oxidizing environment existing in vivo, and the tissue injected with the hydrogel was collected on day 3, day 7 and day 14, followed by histological analysis.

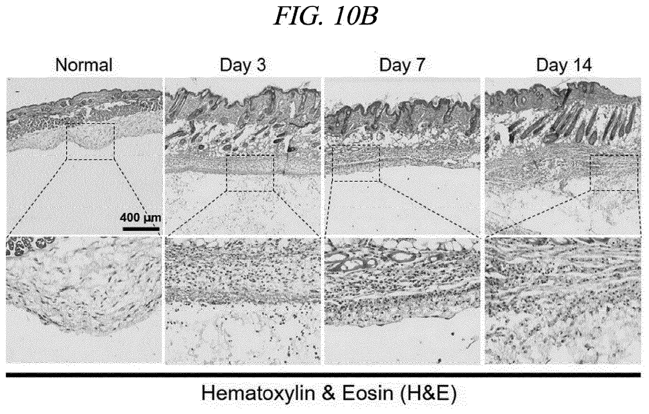

As a result, it was confirmed by H&E staining that the tissue injected with the hydrogel was not significantly different from the normal tissue and no serious immune response occurred. Also, it was confirmed that the adjacent cells were introduced into the hydrogel (see **FIG. 10B**).

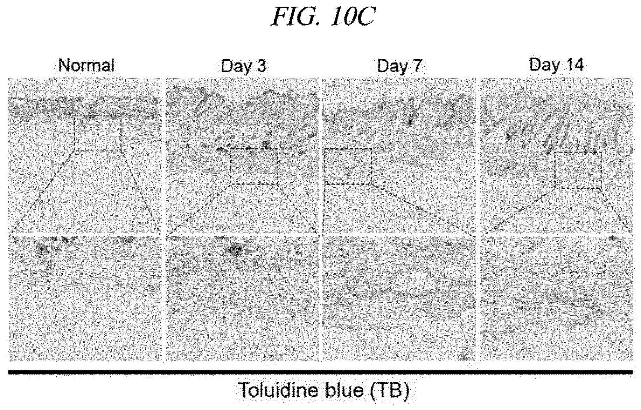

It was confirmed by TB staining that mast cells were hardly observed in the tissue injected with the hydrogel similarly to the normal tissue and an immune response to a foreign material did not occur (see **FIG. 10C**).

Therefore, the Pec-PG hydrogel has a very low possibility of inducing an immune response in the tissue.

### Example 11. Evaluation of safety after intake of gallol group-modified pectin hydrogel

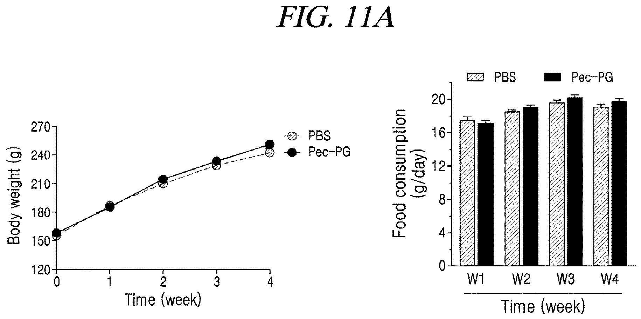

To evaluate the safety after intake of the Pec-PG hydrogel due to characteristics of the product applied to the oral cavity, each of the 2% (w/v) Pec-PG pre-gel solution and the PBS buffer was administered orally to rats every day for 28 days, and the rats' body weight and the amount of feed consumed were periodically measured and compared during the breeding period (see **FIG. 11A**).

As a result, there was no significant difference in body weight and feed weight between the group ingesting the PBS buffer and the group ingesting the Pec-PG solution.

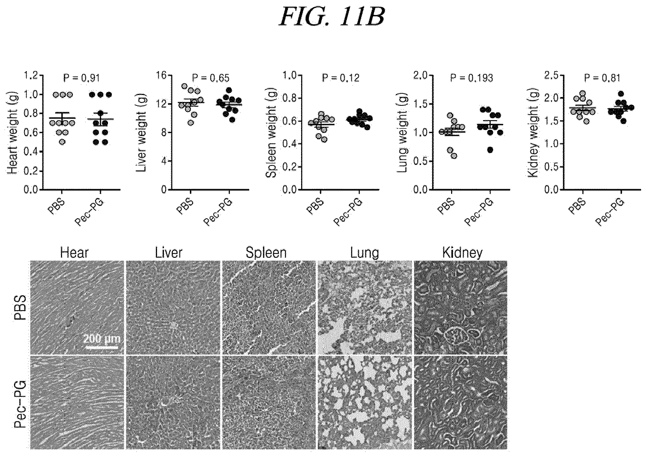

Also, on day 28 after intake of the Pec-PG hydrogel, major organs (heart, liver, spleen, lung, kidney) of the rats were extracted, weighed, and histologically analyzed by H&E staining (see **FIG. 11B**).

As a result, there was no significant difference in organ weight between the group ingesting the PBS buffer and the group ingesting the Pec-PG hydrogel (top), and the histological analysis also confirmed that no tissue damage or immune response occurred (bottom).

Therefore, the Pec-PG hydrogel is expected to be applied without any significant problem with safety when applied to humans.

### Example 12. Measurement of tissue adhesion of gallol group-modified pectin hydrogel

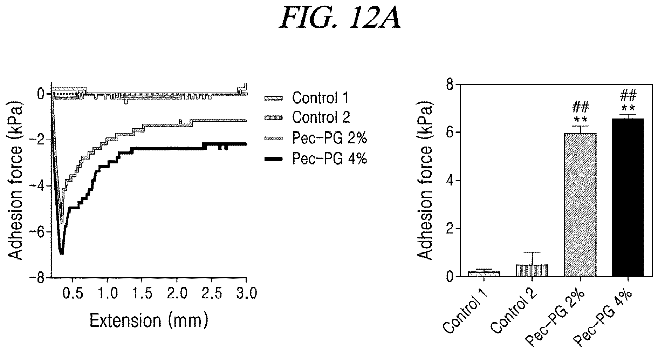

The tissue adhesion was compared between the 2% (w/v) and 4% (w/v) Pec-PG hydrogels and commercial artificial saliva products Control 1 (product name: Xerova; Kolmar Co., Ltd.) and Control 2 (product name: Biotene; GlaxoSmithKline Plc.) by a tack test in a pig tongue tissue (see **FIG. 12A****).**

After the pig tongue tissue was attached to a plate and a probe of a rheometer, the Pec-PG hydrogel crosslinked through spontaneous oxidation or conventional artificial saliva was applied between the tissues to attach the tissues to each other. Then, the detachment force of the tissues was measured while pulling the tissues.

The adhesion of about 0.2 kPa to about 0.5 kPa was measured from Control 1 and Control 2, which confirmed that the tissue adhesion was very low. However, it was confirmed that the Pec-PG hydrogel had an excellent tissue adhesion of about 6 kPa to about 6.5 kPa.

The adhesion was compared between the 2% (w/v) and 4% (w/v) Pec-PG hydrogels and the commercial artificial saliva products Control 1 (product name: Xerova; Kolmar Co., Ltd.) and Control 2 (product name: Biotene; GlaxoSmithKline Plc.) in a living rat tongue tissue.

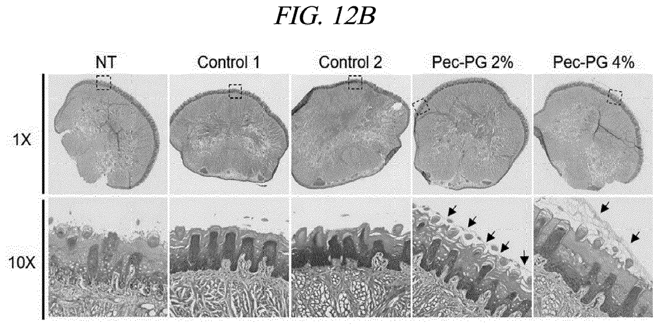

The liquid Pec-PG solution was sprayed onto the rat tongue tissue and attached thereto by inducing crosslinking through in vivo natural oxidation without additional oxidizing agent treatment, and the tissue was collected 30 minutes later, followed by histological analysis (see **FIG. 12B**).

It was confirmed that Control 1 and Control 2 were not left on the tongue tissue, whereas the 2% (w/v) Pec-PG hydrogel was coated and left thin (arrows) and the 4% (w/v) Pec-PG was coated and left thicker (arrows).

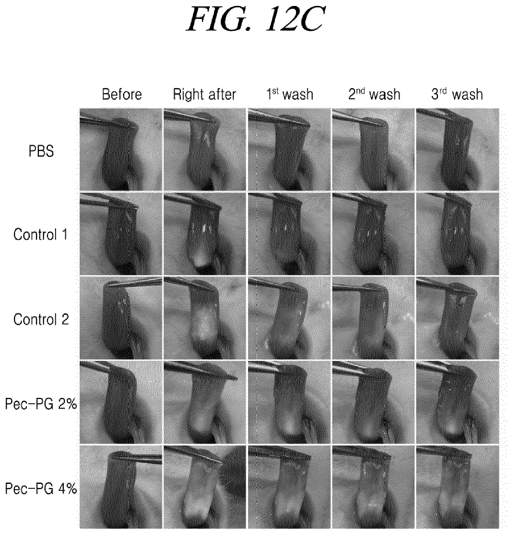

**A** hydrogel mixed with a fluorescent material was coated onto the rat tongue tissue, washed with the PBS buffer, and the adhesion was measured under conditions mimicking an actual oral cavity environment where saliva is present (see **FIG. 12C****).**

It was confirmed that a fluorescence signal disappeared in Control 1 and Control 2 as the number of washing with the PBS buffer increased, whereas a fluorescence signal was maintained and observed well in the 2% (w/v) and 4% (w/v) Pec-PG hydrogels even when washing with the PBS buffer was performed 3 times.

Therefore, due to the excellent adhesion, the Pec-PG hydrogel can be attached and maintained for a longer time in mucosal tissues, particularly an oral cavity environment with a lot of movements and stimulation, such as food intake and mastication. Therefore, the Pec-PG hydrogel is expected to continue moisture retention and drug delivery effects in the oral cavity for a longer time than the conventional materials.

### Example 13. Analysis of moisturizing ability of gallol group-modified pectin hydrogel

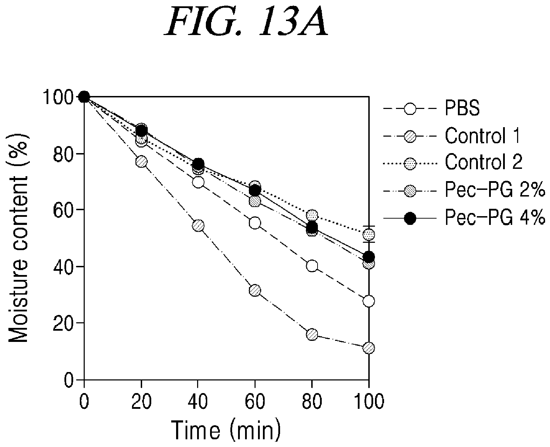

**To** evaluate the moisturizing ability of the Pec-PG hydrogel, the amount of moisture evaporation was compared between the Pec-PG hydrogel and the commercial artificial saliva products Control 1 (product name: Xerova; Kolmar Co., Ltd.) and Control 2 (product name: Biotene; GlaxoSmithKline Plc.) (see **FIG. 13A****).** The Pec-PG hydrogel and the conventional artificial saliva products were coated onto a Petri dish and the weight was measured over time to observe the amount of moisture evaporation.

During a total observation time of 100 minutes, the weight of Control 1 decreased the fastest since moisture of Control 1 evaporated rapidly, and the amount of moisture in the 2% (w/v) Pec-PG, the 4% (w/v) Pec-PG and Control 2 decreased at a similar rate.

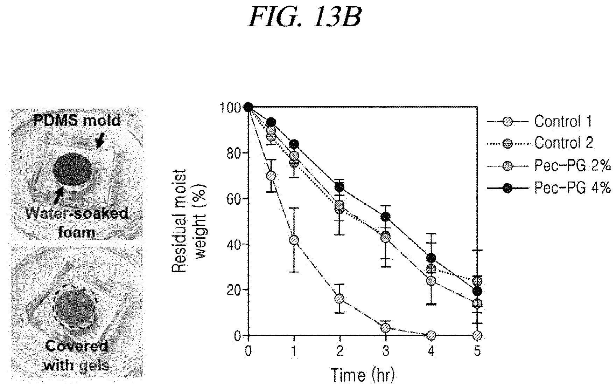

**Also,** to analyze the moisture retention of the Pec-PG hydrogel, a sponge that absorbed water was coated with each of the Pec-PG hydrogel and the conventional artificial saliva products and then the amount of moisture evaporation of the sponge was compared (see **FIG. 13B****).**

**As** a result, moisture evaporated the fastest in the group treated with Control 1, and moisture evaporated at a similar rate in the groups treated with Control 2, the 2% (w/v) Pec-PG hydrogel, and the 4% (w/v) Pec-PG hydrogel.

Therefore, it was confirmed that the Pec-PG hydrogel has the same or greater moisturizing ability than the conventional artificial saliva products.

As confirmed in the previous example, the Pec-PG hydrogel having remarkable tissue adhesion as well as excellent moisturizing ability compared to the conventional artificial saliva products having poor tissue adhesion is highly expected to go beyond the limitations of the conventional products and to be developed as a new artificial saliva for treating mouth dryness.

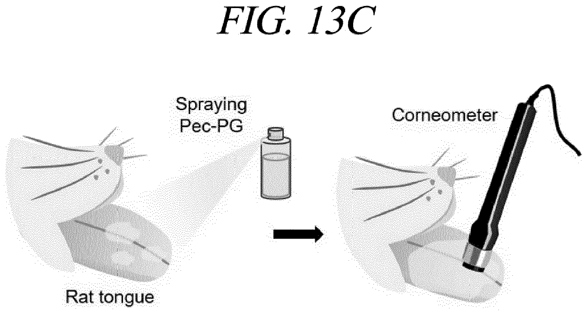

**To** compare the moisturizing ability in the actual oral tissue, the commercial artificial saliva products Control 1 (product name: Xerova; Kolmar Co., Ltd.) and Control 2 (product name: Biotene; GlaxoSmithKline Plc.), and the Pec-PG hydrogel were sprayed onto the living rat tongue tissue. As for the Pec-PG hydrogel, crosslinking was induced through natural oxidation and then, the Pec-PG hydrogel was attached to living rat tongue tissue and the moisture content of the rat tongue tissue was compared by using a corneometer (see **FIG. 13C**). The rat tongue was dried in the same manner for accurate analysis and then treated with 2% (w/v) Pectin, the 2% (w/v) Pec-PG hydrogel, and the conventional artificial saliva, followed by measurement of the moisture content.

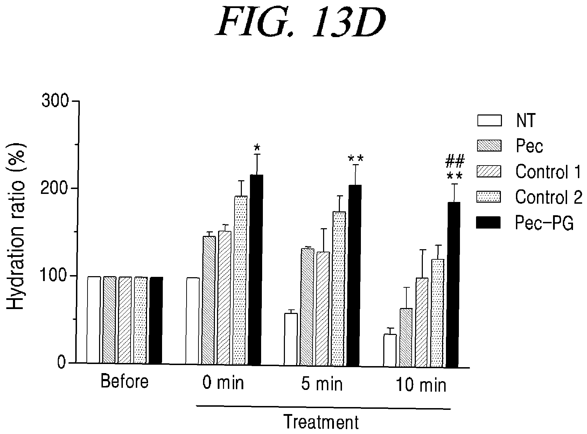

**As** a result of the analysis, it was confirmed that the tongue tissue coated with the Pec-PG hydrogel best maintained the moisture content (see **FIG. 13D**).

Therefore, it was confirmed that when applied to the actual oral tissue, the Pec-PG hydrogel has superior moisturizing ability and excellent functionality as artificial saliva compared to the conventional artificial saliva products.

### Example 14. Analysis of physical properties of gallol group-modified pectin hydrogel patch

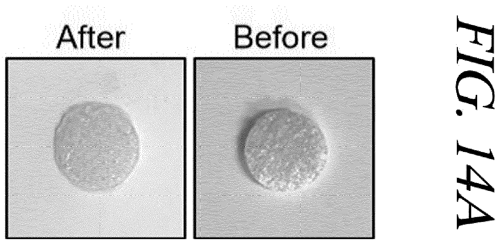

The Pec-PG solution was poured into a mold having a desired shape and size (Example: a 8 mm diameter disc) and frozen at -80°C and freeze-dried to fabricate a Pec-PG patch (see **FIG. 14A****;** Before). Crosslinking was induced by spraying 4.5 mg/ml NalO₄ as an oxidizing agent onto the fabricated Pec-PG patch (see **FIG. 14A****;** After).

After crosslinking was induced by spraying 4.5 mg/ml NalO₄ as an oxidizing agent onto the Pec-PG patches fabricated at two different concentrations (2% (w/v), 4% (w/v)), the swelling ratio of each Pec-PG hydrogel patch was measured.

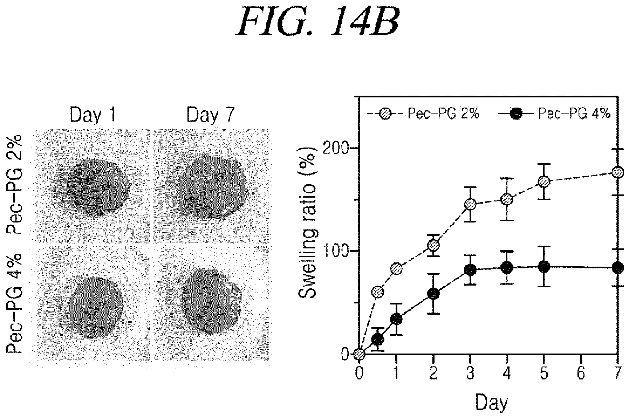

When kept under physiological conditions (37°C in PBS buffer) for 7 days, the 2% (w/v) Pec-PG hydrogel patch showed a higher swelling ratio than the 4% (w/v) Pec-PG hydrogel patch (see **FIG. 14B****).**

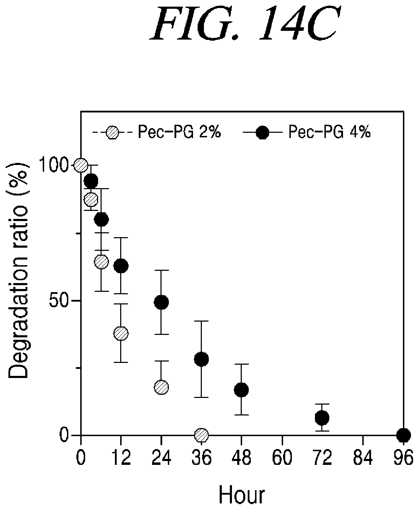

To compare the degradation pattern of the Pec-PG hydrogel patch, the hydrogel patch was treated with a pectinase. When treated with the same concentration of the pectinase (2 U/sample), the 4% (w/v) hydrogel patch was degraded later due to a higher degree of crosslinking of gallol groups (see **FIG. 14C**).

That is, the physical properties, such as swelling ratio and degradation behavior, of the Pec-PG hydrogel patch can be controlled by controlling the concentration of the Pec-PG.

### Example 15. Analysis of porous structure of gallol group-modified pectin hydrogel patch

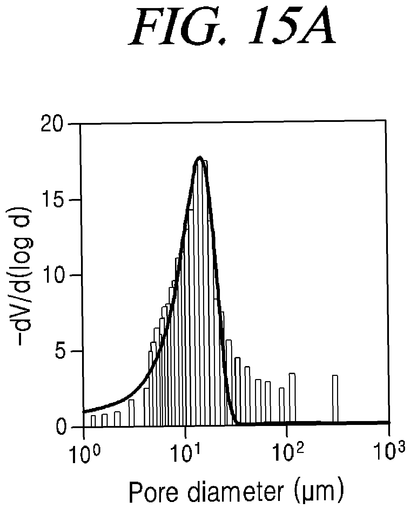

When a pore diameter in the 2% (w/v) Pec-PG hydrogel patch was measured with a porosimeter, a large number of pores had a diameter of about 100 µm (see **FIG. 15A**).

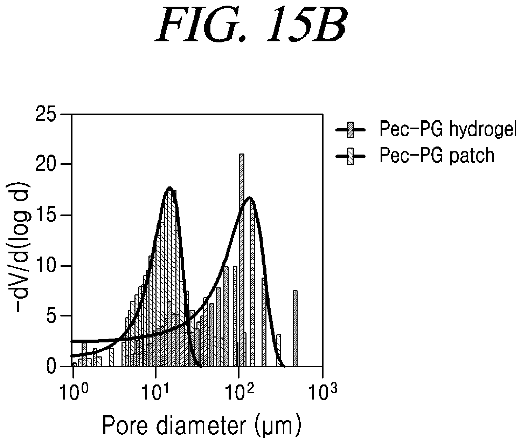

When compared with the pore diameter in the Pec-PG hydrogel, the Pec-PG hydrogel patch had a structure with smaller pores (see **FIG. 15B**).

That is, it seems that the freeze-dried Pec-PG hydrogel in the form of a patch has a denser porous internal structure than the Pec-PG hydrogel crosslinked in a solution, and, thus, the Pec-PG hydrogel patch increases in adhesive surface area. Therefore, the Pec-PG hydrogel patch is expected to have improved mechanical properties and adhesiveness.

### Example 16. Analysis of mechanical properties of gallol group-modified pectin hydrogel patch

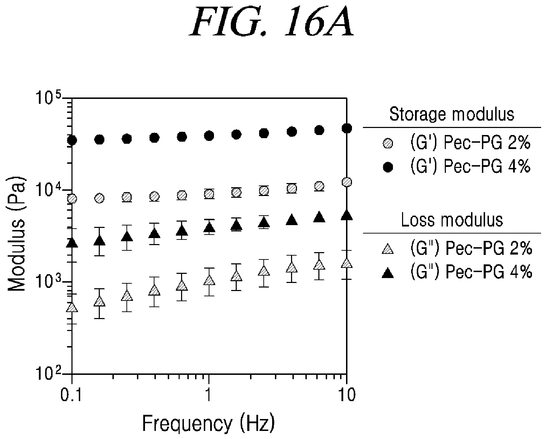

The elastic modulus of each of the Pec-PG hydrogel patches at two different concentrations (2% (w/v), 4% (w/v)) was measured in a frequency sweep mode by using a rheometer (see **FIG. 16A****).** It was confirmed that a stable polymer network structure was formed in each hydrogel patch by confirming that the G' value was higher than the G" value in both the 2% (w/v) and 4% (w/v) Pec-PG hydrogel patches.

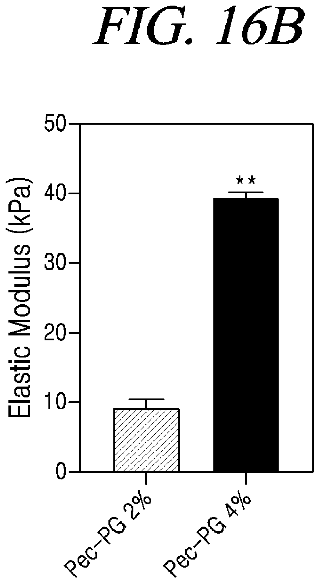

The average elastic modulus of the 2% (w/v) Pec-PG hydrogel patch was about 10 kPa, whereas the average elastic modulus of the 4% (w/v) Pec-PG hydrogel patch was about 40 kPa, which confirmed that the elastic modulus increases in proportion to the concentration of the Pec-PG (see **FIG. 16B****).**

That is, the mechanical properties of the Pec-PG hydrogel patch can be controlled by controlling the concentration of the Pec-PG.

By comparison between the Pec-PG hydrogel in the form of a patch and the hydrogel crosslinked in the Pec-PG solution at the same concentration, it was confirmed that the elastic modulus of the Pec-PG hydrogel patch was significantly increased (the elastic moduli of the 2% (w/v) and 4% (w/v) Pec-PG hydrogels were 1.7 kPa and 4.9 kPa, respectively, and the elastic moduli of the 2% (w/v) and 4% (w/v) Pec-PG hydrogel patches were 10 kPa and 40 kPa, respectively). Thus, it is expected that the Pec-PG hydrogel patch can be stably maintained even in an in vivo environment, such as oral cavity with high fluidity and large physical deformation.

### Example 17. Measurement of tissue adhesion of gallol group-modified pectin hydrogel patch

The tissue adhesion was compared between the 2% (w/v) and 4% (w/v) Pec-PG hydrogel patches by a tack test. The adhesion to the pig gum tissue was evaluated.

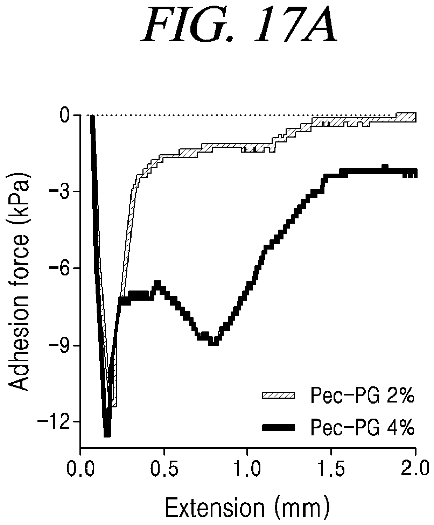

After the pig gum tissue was attached to a plate and a probe of a rheometer, the Pec-PG hydrogel patch crosslinked through natural oxidation was applied between the tissues to attach the tissues to each other. Then, the detachment force of the tissues was measured while pulling the tissues (see **FIG. 17A****).**

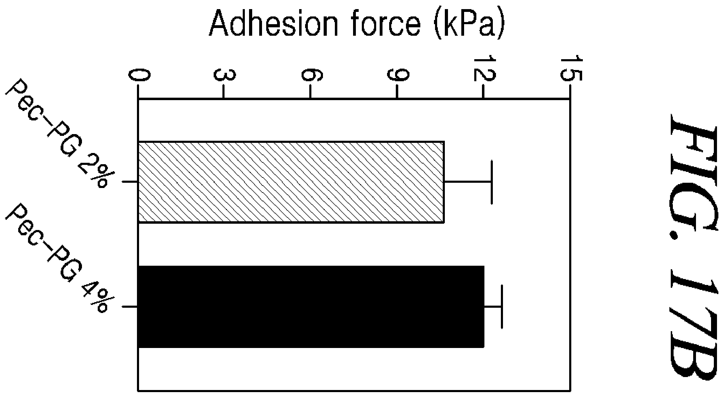

It was confirmed that both the 2% (w/v) and 4% (w/v) Pec-PG hydrogel patches had excellent tissue adhesion at a level of about 10 kPa to about 12 kPa (see **FIG. 17B**).

Therefore, due to the excellent tissue adhesion, the Pec-PG hydrogel patch can be attached and maintained for a longer time in an oral cavity environment with a lot of movements and stimulation, such as food intake and mastication. Therefore, the Pec-PG hydrogel patch is expected to continue a drug delivery effect in the oral cavity for a longer time than the conventional materials.

### Example 18. Analysis of drug release pattern of gallol group-modified pectin hydrogel patch

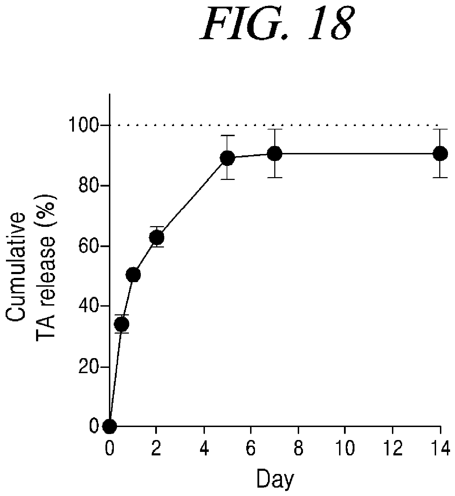

**To** verify sustained drug release delivery of the Pec-PG hydrogel patch, the release pattern of triamcinolone acetonide (TA), a stomatitis drug, was analyzed. The release pattern of TA was observed during treatment with a pectinase (0.1 U/sample) under physiological conditions (37°C in PBS buffer) (see **FIG.** 18).

When the drug release pattern was checked by HPLC, a sustained release behavior of TA from the Pec-PG hydrogel was observed.

Therefore, the Pec-PG hydrogel patch loaded with a drug can be used to deliver a drug effective for stomatitis. Also, it is expected that a continuous treatment can be induced.

### Example 19. Analysis of antioxidant function of gallol group-modified pectin hydrogel and patch formulation

**A** test was conducted to check whether the Pec-PG hydrogel and patch are able to scavenge reactive oxygen species (ROS) and various harmful radicals with oxidized derivatives of the modified gallol groups.

To verify the antioxidant function of the Pec-PG hydrogel and patch, the effect of scavenging 2,2'-azino-bis(3-ethylbenzothiazoline-6-sulfonic acid)(ABTS) radicals and hydroxyl radicals was analyzed.

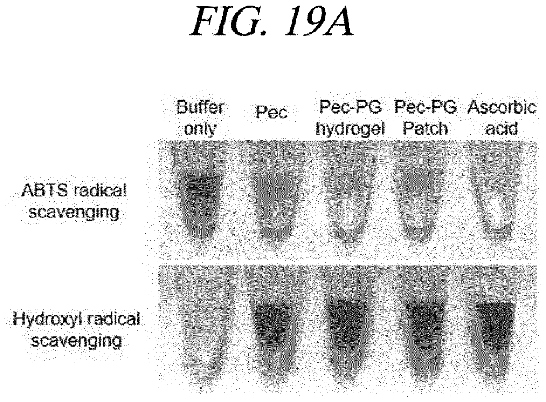

For antioxidant test, an ABTS solution that changes from blue-green to transparent when reacted with a sample with high antioxidant activity was used (see **FIG. 19A****;** top). As a negative control, an ABTS solution as a buffer was used, and as a positive control, ascorbic acid known as vitamin C with high antioxidant activity was used.

Further, a Fenton reaction reagent in which H₂O₂ and Fe²⁺ ions react to generate Fe³⁺ ions and hydroxyl radicals that induce DNA damage and cell death was used to check whether the above-described reaction is inhibited in the presence of the Pec-PG hydrogel and patch (see **FIG. 19A****;** bottom). As a negative control, only a buffer was used, and as a positive control, ascorbic acid was used.

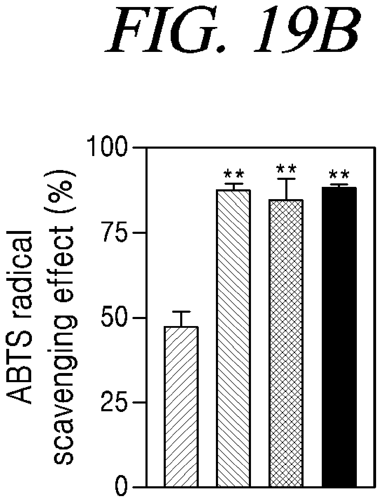

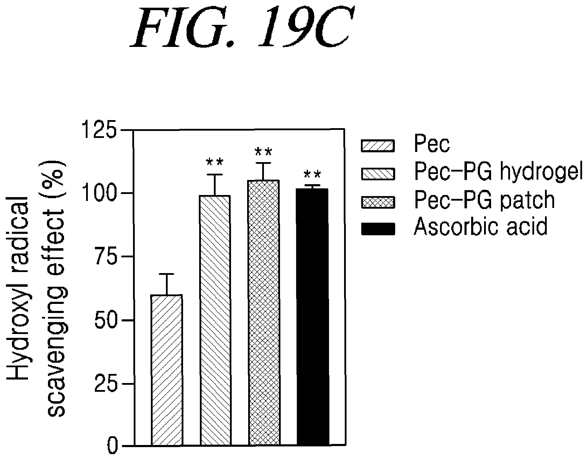

The 4% (w/v) Pec-PG hydrogel and patch were put in a solution prepared by using the ABTS solution and the Fenton reaction and incubated for 30 minutes, and the hydrogel and patch were removed to measure the color of the remaining solution by UV-vis spectroscopy and check whether ABTS radicals and hydroxyl radicals are removed (see **FIG. 19B** and **FIG. 19C**). As a result, it was confirmed that both the Pec-PG hydrogel and the Pec-PG patch had as much antioxidant activity as the positive control, ascorbic acid.

Therefore, the Pec-PG hydrogel and patch are expected to contribute to reducing tissue damage due to their excellent antioxidant function as well as their function as a sustained drug release system.

### Example 20. Evaluation of therapeutic efficacy of gallol group-modified pectin hydrogel patch on rat diabetic stomatitis

After the rats were fasted for 24 hours, diabetes was induced by intraperitoneal injection of streptozotocin (65 mg/kg). After 48 hours, a fasting blood sugar level was measured as 200 mg/dL or more.

**A** stomatitis model was created by treating the gums of the diabetic rats with 80% acetic acid to induce a tissue damage having a diameter of about 4 mm. The Pec-PG hydrogel patch loaded with triamcinolone acetonide (TA), a stomatitis drug, was applied to the stomatitis site on the day of inducing tissue damage and day 4 through natural oxidation without an oxidizing agent to confirm the effects of gum regeneration and inflammation treatment (see **FIG. 20A****).**

The following six groups were used for test.
1) No treatment
2) Apply TA drug only (TA only)
3) Apply Pec-PG patch only (Patch only)
4) Apply commercialized drug loaded with 25 µg of TA (Control: product name: Aftach; Dong-Wha Pharm. Ind. Co., Ltd.)
5) Pec-PG patch loaded with 12.5 µg of TA (Patch+TA(1x))
6) Pec-PG patch loaded with 25 µg of TA (Patch+TA(2x))

The Pec-PG patch loaded with TA was attached and an inflammation stie was visually observed on day 4 and day 7. When the Pec-PG patches (Patch+TA(1x), Patch+TA(2x)) loaded with TA were applied, the area of a damage site was decreased the most and the effect of tissue regeneration was also enhanced the most (see **FIG. 20B** and **FIG. 20C****).**

To evaluate the degree of tissue regeneration in the stomatitis-induced damage site, H&E staining, immunostaining for epithelial tissue markers (cytokeratin 5; CKS, cytokeratin 13; CK13) and staining image-based quantitative analysis were performed on day 7 after treatment. Accordingly, it was confirmed that the regeneration of epithelial tissue was greatly enhanced in the group (Patch+TA(1x), Patch+TA(2x)) to which TA was delivered using the Pec-PG patch (see **FIG. 20D** to **FIG. 20F**).

Through immunostaining for CD11b, a leukocyte marker, a number of inflammatory cells in a tissue damage site were observed in No treatment group, the group treated with the Pec-PG patch or TA only, and the group applied with a conventional product. However, a significantly smaller number of inflammatory cells were observed in the group (Patch+TA(1x), Patch+TA(2x)) to which TA was delivered using the Pec-PG patch (see **FIG. 20D** and **FIG. 20G****).**

Accordingly, it was confirmed that the Pec-PG patch was well fixed to the inflammation stie, protected the damaged tissue from external stimuli, and promoted the regeneration of epithelial tissue due to its antioxidant function and effective drug delivery function.

### Comparative Example 1. Comparison of mucosal adhesion between gallol group-modified pectin hydrogel and prior art

Analysis was conducted to verify that the Pec-PG hydrogel developed in the present disclosure has better mucoadhesion than HA-PG and Chi-CA hydrogels developed in a prior art document (Korean Patent No. 10-1942220). Like Pec-PG, HA-PG and Chi-CA are materials used in previous studies to induce crosslinking through natural oxidation.

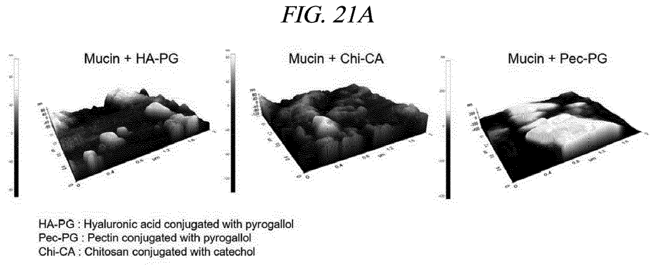

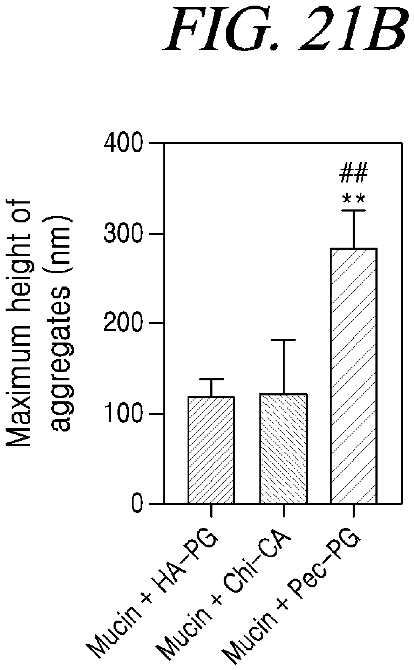

The reactivity and interaction between mucin protein and each derivative were analyzed by atomic force microscopy (AFM) (see **FIG. 21A****).** It was confirmed that larger aggregates were formed on the surface when the mucin protein solution was mixed with the Pec-PG solution than when the mucin protein solution was mixed with the HA-PG solution or the Chi-CA solution (see FIG. 21B).

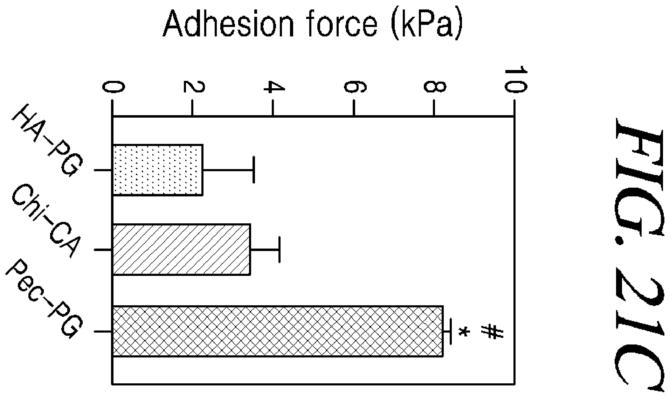

Based on these results, the mucoadhesion in the mucosal tissue of the pig gum was actually compared among the Pec-PG hydrogel patch, the HA-PG hydrogel patch, and the Chi-CA hydrogel patch by a tack test (see **FIG. 21C****).** It was found that the Pec-PG hydrogel patch had a mucoadhesion of about 8.3 kPa, whereas each of the HA-PG hydrogel patch and the Chi-CA patch had a mucoadhesion of about 2.3 kPa and about 3.5 kPa, respectively. Therefore, it was confirmed that the Pec-PG hydrogel patch had the best mucoadhesion.

### Comparative Example 2. Comparison of fabrication method of gallol group-modified pectin hydrogel patch and prior art

According to a technology of fabricating a patch disclosed in a prior art document (Korean Patent Laid-open Publication No. 10-2018-0127634 No., etc.), crosslinking of Pec-PG was induced by using 4.5 mg/ml NalO₄ as an oxidizing agent, followed by freeze-drying to fabricate a Pec-PG patch (post-crosslinking freeze-dried patch).

The Pec-PG patch according to the present disclosure was fabricated by pouring a Pec-PG solution into a mold and freeze-drying it immediately without crosslinking (freeze-dried patch prior to crosslinking).

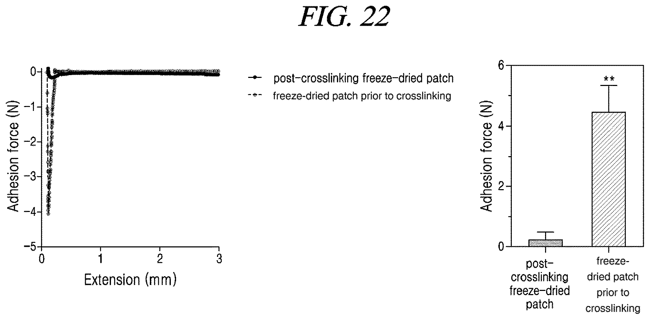

After crosslinking was induced by using 4.5 mg/ml NalO₄ as an oxidizing agent, the patch was attached to a plate and a probe of a rheometer and the tissue adhesion of each Pec-PG patch was compared by a tack test. As a result, it was confirmed that the freeze-dried patch prior to crosslinking (the patch fabricated according to the present disclosure) had a significantly better adhesion than the post-crosslinking freeze-dried patch (the patch fabricated by the general method according to the prior art document) (see **FIG. 22**).

## Claims

1. A pectin derivative modified with a gallol group derivative (Pec-PG).

2. The pectin derivative of Claim 1,
wherein the gallol group derivative is one selected from the group consisting of 5-hydroxydopamine, tannic acid, gallic acid, epigallocatechin, epicatechin gallate, epigallocatechin gallate, 2,3,4-trihydroxybenzaldehyde, 2,3,4-trihydroxybenzoic acid, 3,4,5-trihydroxybenzaldehyde, 3,4,5-trihydroxybenzamide, 5-tert-butylpyrogallol, and 5-methylpyrogallol.

3. A hydrogel prepared by oxidizing the pectin derivative of Claim 1.

4. The hydrogel of Claim 3,
wherein the oxidation is performed by adding an oxidizing agent, an enzyme, or a pH adjustor.

5. The hydrogel of Claim 4,
wherein the oxidizing agent is periodate salt or hydrogen peroxide.

6. The hydrogel of Claim 4,
wherein the enzyme is one selected from the group consisting of peroxidase, horseradish peroxidase, and tyrosinase.

7. The hydrogel of Claim 4,
wherein the pH adjustor is one selected from the group consisting of sodium hydroxide, lithium hydroxide, potassium hydroxide, rubidium hydroxide, cesium hydroxide, magnesium hydroxide, calcium hydroxide, strontium hydroxide, and barium hydroxide.

8. The hydrogel of Claim 3,
wherein the oxidation is performed through natural oxidation.

9. The hydrogel of Claim 3,
wherein an application site of the hydrogel includes oral mucosa, nasal mucosa, ophthalmic mucosa, gastric mucosa, intestinal mucosa, bronchial mucosa, heart, lung, or urinary tract.

10. The hydrogel of Claim 3,
wherein the hydrogel is used to treat mucosal tissue diseases.

11. The hydrogel of Claim 10,
wherein the mucosal tissue diseases include oral mucositis, oral cancer, stomatitis, vitiligo, hand-foot-and-mouth disease, oral tuberculosis, oral candidiasis, mucosal wounds, periodontitis, aphthous ulcer, Behcet's syndrome, lichen planus, or herpetic stomatitis.

12. The hydrogel of Claim 3,
wherein the hydrogel is used for controlled drug delivery.

13. The hydrogel of Claim 12,
wherein the drug includes one or more selected from the group consisting of DNA, mRNA, siRNA, miRNA, antisense oligonucleotide, antihistamine, proteins (growth factors), corticosteroid-based steroids, immune cell activators, doxorubicin, daunomycin, epirubicin, anthracyclines, paclitaxel, docetaxel, cabazitaxel, taxanes, curcumin, campotecin, berberine, evodiamine, matrine, piperine, sanguinarine, tetrandrine, talicarpine, alkaloids, vinblastine, vincristine, vindesine, vinca alkaloids, cisplatin, carboplatin, platinums, 5-fluorouracil, capecitabine, methotrexate, antimetabolites, irinotecan, topotecan, etoposide, teniposide, etoposide, topoisomerase inhibitors, bleomycin, actinomycin, mitomycin, antitumor antibiotics, cyclophosphamide, mechlorethamine, chlorambucil, melphalan, alkylating agents, azacitidine, azathioprine, cytarabine, doxifluridine, therapeutic antibodies, antibiotics, antibacterial agents, antiviral agents, anti-inflammatory agents, protein drugs, growth factors, cytokines, peptide drugs, and anesthetics.

14. A patch comprising the pectin derivative of Claim 1.

15. The patch of Claim 14,
wherein the patch is prepared by freeze-drying Pec-PG before crosslinking.

16. A method of preparing pectin modified with a gallol derivative, comprising:
a process of reacting pectin, EDC, NHS, and a gallol derivative in a predetermined ratio.

17. The method of Claim 16,
wherein the ratio of pectin:EDC:NHS:PG is 1:2:2:2.
